# EUROPEAN PATENT SPECIFICATION

(11) **EP 0 617 286 B1**
(45) Date of publication and mention of the grant of the patent: **22.12.1999**
(21) Application number: 94850024.4
(22) Date of filing: 16.02.1994
(51) Int. Cl.: G01N 33/546, G01N 33/543, G01N 33/58, G01N 33/78, G01N 33/68, G01N 21/64

(54) **Biospecific solid phase carrier**
Biospezifischer Festphasenträger
Support biospécifique en phase solide

(30) Priority: 18.03.1993 FI 931198
(43) Date of publication of application: 28.09.1994
(73) Proprietor: WALLAC OY, 20101 Turku (FI)
(72) Inventor: Iitiä, Antti Juhana, SF-21250 Masku (FI); Lövgren, Timo Nils-Erik, SF-Fin-21610 Kirjala (FI); Pettersson, Kim Sverker Immanuel, SF-20810 Turku (FI)
(74) Representative: Larsson, Kjell

(56) References cited:
- EP-A- 0 296 136
- WO-A-88/02784
- WO-A-92/12255
- CLINICAL CHEMISTRY, vol. 38,no. 10, 1992 WASHINGTON, DC, pages 2038-2043, XU ET AL. 'Simultaneous quadruple-label fluorometric immunoassay ....'
- CRC CRITICAL REVIEWS IN ANALYTICAL CHEMISTRY, vol. 18,no. 2, 1987 CLEVELAND, OHIO, pages 105-154, SOINI ET AL. 'Time-resolved fluorescence of lanthanide probes ....'

## Description

This invention relates to a biospecific assay method which allows the simultaneous determination of a plurality of analytes in the same sample.

### BACKGROUND OF THE INVENTION

Microparticles can be employed as a solid phase carrier in various bioaffinity assays. In such assays it is often useful or necessary to perform a plurality of simultaneous assay reactions, thus eliminating repetition of parallel assay steps. One possibility to perform multiparameter analysis is offered by the use of microparticles as a solid phase carrier (Soini E., US 5,028,545). In such a system specific probes are linked to said particles, whereafter particles embodying different specificities may be mixed. The mixture can thus be used as a solid phase carrier in bioaffinity assays. When taking measurements after the completion of the reaction it is essential to be able to identify as many particle categories with different specificities as possible. For the purpose of identification such properties as the size of the particles (McHugh, T.M. et al., J. Immunol. Methods 1986; 95: 57 - 61) may be utilized, or coloured particles (Streefkerk J.G., Kors, N., Boden, D. Protides Biol. Fluids 1976; 24: 811 - 814) or particles labelled with various fluorescent molecules (Dean, K.J. et al. Clin. Chem. 1983; 29: 1051 - 1056) may be employed.

In bioaffinity reactions two biological molecules are capable of binding with great accuracy even in the presence of other molecules. Such molecules capable of binding to each other are e.g. antibodies and their corresponding antigens, single-stranded DNA molecules and their corresponding nucleic acid sequences, and receptors and the molecules that specifically bind to them. All these reactions may be given the common denomination of bioaffinity reactions.

Bioaffinity reactions are exploited in various practical applications and in assays in research. A known biomolecule, which may be purified from biological material (e.g. an antibody) or synthesized chemically (e.g. an oligonucleotide DNA) can be labelled in such a manner as to allow detection after the completion of the bioaffinity reaction. At present the major applications of bioaffinity reactions include immunoassays and nucleic acid hybridization assays, which are used to assay various components from e.g. blood. Assays based on receptors as biological reactants are important in the search for new medicinal substances.

Assays performed on blood or its components may be divided in groups relevant to certain diseases or medical operations. In multiparameter assays the analytes belonging to these groups, so-called panels, could be assayed simultaneously in the same sample. This would enable economies to be made in the costs involved in testing, which would result in an increase in the number of tests that could be performed, and consequently in more reliable diagnoses. Multiparameter assays would also require considerably less work in performing the tests, and would consequently yield faster and more reliable results.

Natural objects for assays employing DNA as the biological reactant would be viruses and inherited diseases, which are caused by mutations in cellular DNA. Especially inherited diseases may present multiple DNA mutations, and it would be imperative in the diagnosis for inherited diseases to be able to determine a number of DNA mutations simultaneously in the same sample. This would be especially useful in the search for carriers of these mutations in large studies covering whole populations.

Because of the minute amount of the DNA to be analyzed the gene domain will often have to be amplified from the sample before the identification of the domain or the mutations. Several gene domains may be amplified simultaneously in the same reaction. Multiparameter assays may be advantageously applied to the analysis of such reaction mixtures containing several gene domains.

In multiparameter assays labels can be used whose signals, after the completion of the bioaffinity reaction, can be resolved on the basis of e.g. wavelength or the decay time of the signal. Such assays have predominantly employed different radioactive isotopes (Morgan C.R., Proc. Soc. Exp. Biol. Med. 1966; 123: 230 - 233; Wians, F.H., Dev, J., Powell, M.M., Heald, J.I., Clin. Chem. 1986; 32: 887 - 890), enzyme labels (Dean, K.J., Thompson, S.G., Burg, J.F., Buckler, R.T., Clin. Chem. 1983; 29: 1051 - 1056; Bates, D.I., Bailey, W.R., International Patent Application WO89/06802), and labels emitting short-lived fluorescence. Such labels however often present problems due to overlapping of the wavelengths of the signals to be measured (isotopes, fluorescent molecules), to the proximity of the excitation and emission wavelengths (fluorescent molecules), to different optimal conditions (enzyme labels) or to background fluorescence of the material (labels emitting short-lived fluorescence). Such systems have at best allowed only two simultaneous determinations.

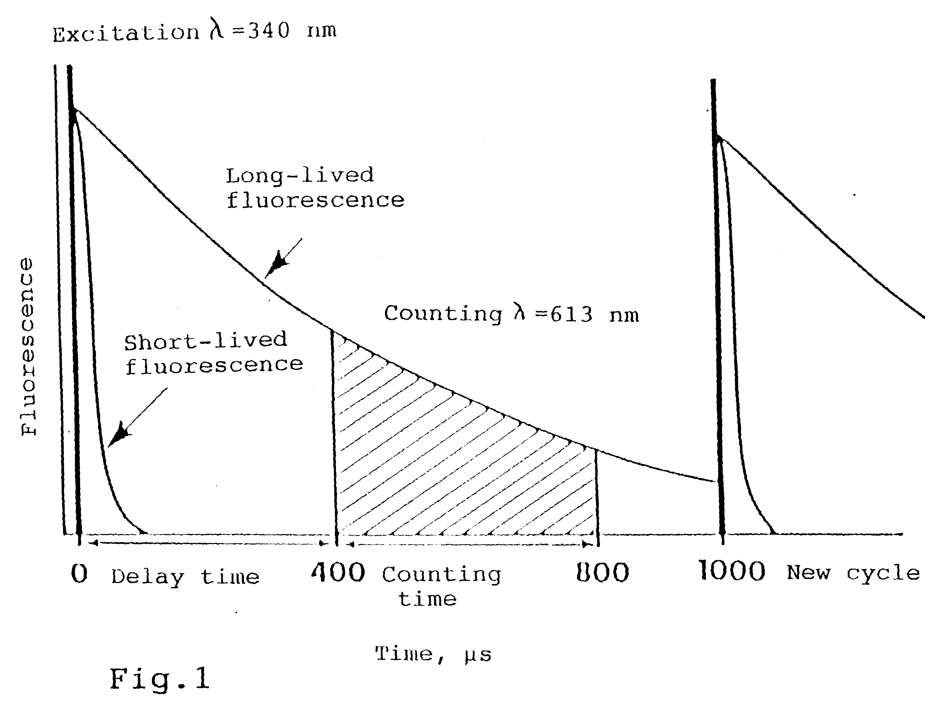

Systems using label technology based on time-resolved fluorescence take advantage of the long-lived fluorescence of rare earth metals (Figure 1; Soini, E. and Lövgren, T., CRC Critical Reviews in Analytical Chemistry 1987; 18(2): 105 - 154). The metal is complexed to an organic molecule capable of absorbing energy during the excitation of the complex. On the other hand, the said ligand must also be able to release the excitation energy to the metal ion in the complex. The most useful earth metals in time-resolved labels are Eu, Sm, Tb and Dy. The energy levels of the electrons of the said rare metals are the best suited to absorb the excitation energy, and on the other hand, energy transitions which decrease long-lived fluorescence are slight in the complexes of these earth metals. The energy transitions to the ground state in these earth metals result in fluorescence consisting of several narrow bands of different wavelengths. Some electronic transitions are however, on transition to the ground state, preferred compared to others, and consequently the majority of the observed fluorescence has components of distinct wavelength. On simultaneous measurement in the bioaffinity assays of the reactants containing these time-resolved labels the effect of the minor components on measurement results can be easily eliminated by computational methods. In multiparameter assays using time-resolved labels as many as four different biomolecules have been measured simultaneously with required sensitivity (Xu, Y.Y., Pettersson, K., Blomberg, K., Hemmilä, I., Mikola, H. and Lövgren, T., Clin. Chem. 1992; 38/10: 2038 - 2043).

An alternative possibility as the principle for multiparameter assays is available in the use of different solid phase carriers for the distinguishing of specific probes in bioaffinity assays. A solid phase carrier can be divided into domains each containing a probe of a determined specificity; different solid phase carriers can be used simultaneously in the assay, or the solid phase carrier can be distributed in different categories on the basis of a label (Soini, E., US 5,028,545). In the last example each solid phase category has a probe of different specificity bound to the surface. The application of this alternative principle to multiparameter assays presents several advantages because each component of the multiparameter system can be manufactured separately, and then combined. This is important from the point of view of production technology.

### SUMMARY OF THE INVENTION

This invention relates to a biospecific multiparameter assay method employing microparticles distributed in different categories and representing different analytes, in which method the microparticles are labelled with a fluorescent molecule (1) and in which method microparticles of different categories are coated with bioaffinity reactants A binding different analytes. In the said method the microparticles of different categories are mixed, the sample to be analyzed is added, and finally the bioaffinity reactants B labelled with a fluorescent molecule (2) are added either immediately or after the completion of the reaction between the analyte molecule and the bioaffinity reactant A. The fluorescent molecules (1, 2) are excited and the fluorescent emissions are quantitated for the identification of the microparticle category and for the measurement of the contents of different analytes. The method according to this invention is characterized by the labelling of the microparticles and of the bioaffinity reactants B with different molecules emitting long-lived fluorescence (1, 2).

The said biospecific multiparameter assay method is performed either in one step with all the components of the reaction present simultaneously, or in two steps, in which case labelled bioaffinity reactant B is added after the completion of the reaction between bioaffinity reactant A and the analyte molecule.

According to one useful variant method time-resolved fluorescence, i.e. a molecule emitting long-lived fluorescence, is used for the identification of the microparticle category as well as for the labelling of the bioaffinity reactant B.

### BRIEF DESCRIPTION OF THE DRAWINGS

Figure 1 shows the fluorescense as function of time for long-lived and short-lived fluorescence
Figure 2 shows the dose-response curve of europium where the standards (EuCl₃) were made out in a fluorescence-enhancing solution and the fluorescence was measured over 1 second with a time-resolved fluorometer
Figure 3 shows the wavelength, delay and intensity profiles of the emission from a mixture of PTA-chelated Eu⁺³, Tb⁺³, Sm⁺³ and Dy⁺³

### DETAILED DESCRIPTION OF THE INVENTION

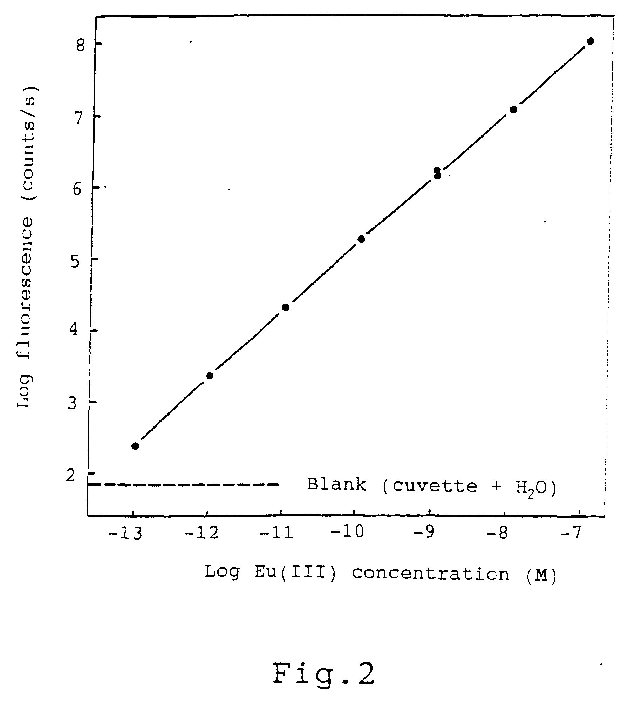

This invention employs time-resolved fluorescence (Soini, E. and Lövgren, T., CRC Critical Reviews in Analytical Chemistry 1987; 18(2): 105 - 154) for the labelling of the said particles or other type of solid phase carrier. Time-resolved fluorescence allows the elimination of the short-lived background fluorescence due to the natural fluorescence of different components of the system. Consequently, when the particles are labelled with different concentrations of the time-resolved label, an extremely low concentration of label can be detected. The sensitivity and the good linearity in the measurement of the time-resolved fluorescence allow the measurement of the time-resolved label over an extremely wide concentration range. Figure 2 shows the dose-response curve of europium where the standards (EuCl₃) were made out in a fluorescence-enhancing solution and the fluorescence was measured over 1 second with a time-resolved fluorometer (1230 Fluorometer, LKB-Wallac, Turku, Finland). A wide concentration range on the other hand enables the identification of a plurality of different categories when differentiating the solid phase carriers. Time-resolved fluorescence technology also allows the use of labels emitting radiation of different wavelengths. (Saarma, M., Järvekulg, L., Hemmilä, I., Siitari, H., & Sinijärv, R., Journal of Virological Methods 1989; 23: 47 - 54; Xu, Y.Y., Pettersson, K., Blomberg, K., Hemmilä, I., Mikola, H. and Lövgren, T., Clin. Chem. 1992; 38/10: 2038 - 2043; Iitiä, A., Liukkonen, L. and Siitari, H., Molecular and Cellular Probes 1992; 6: 505 - 512). This invention also makes use of the combination of the concentrations of these labels emitting different wavelengths, in the labelling of the particles or other types of solid phase carriers.

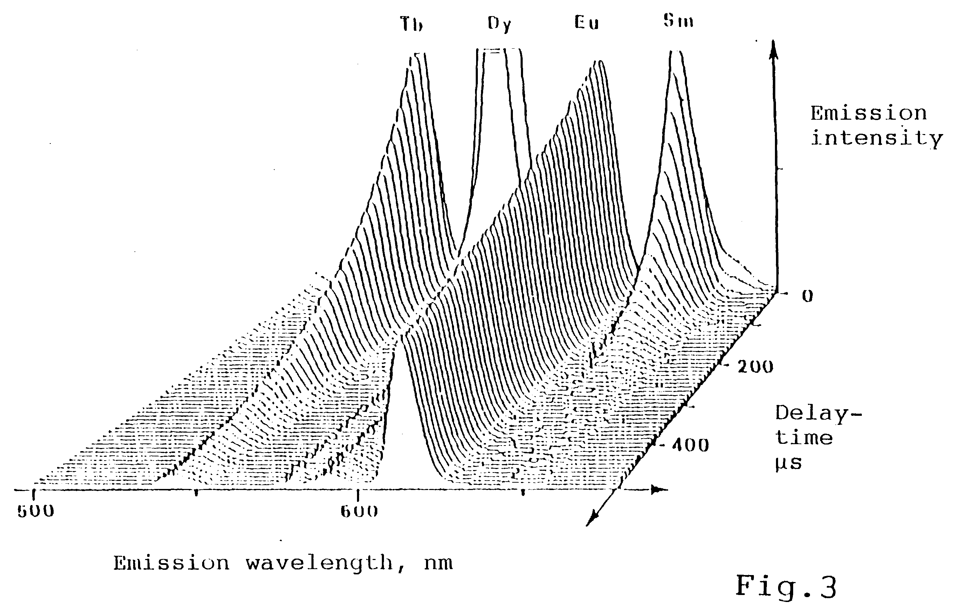

When using the microparticle as a solid phase carrier in the identification of different analytes in a multiparameter assay only one or a few labels are needed for the detection of the actual analyte. When the label technology is based on time-resolved fluorescence which allows the resolution of several labels, the use of a categorizable solid phase carrier will free one or more labels for use in the identification of the categories of the solid carrier. Figure 3 shows the wavelength, delay and intensity profiles of the emission from a mixture of PTA-chelated Eu⁺³, Tb⁺³, Sm⁺³ and Dy⁺³.

The solid phase carrier can be distributed into categories on the basis of either time-resolved labels fluorescing at different wavelengths, on the basis of different concentrations of time-resolved label, on the basis of simultaneous use of time-resolved and short-lived fluorescent labels, or a combination of these. For instance, by using two time-resolved labels fluorescing at different wavelengths, in ten different concentrations, for the identification of the categories of solid phase carrier, one hundred different solid phase carrier categories can be identified.

An extremely great number of identifiable solid phase carrier categories can be incorporated in one multiparameter assay by using microparticles containing fluorescent lanthanide chelates. Different fluorescent molecules can be linked to different or the same microparticles. It is advantageous to label the same microparticles with many different fluorescent molecules because the combinations of the different fluorescent chelates and/or the combinations of their concentrations will result in a great number of possible combinations. The solid phase carrier categories are identified by measuring the fluorescence intensity of the lanthanide chelate/chelates contained in the individual microparticles by using time-resolved fluorescence. After identification, the result of the commonly known specific bioaffinity reaction performed on the particle is determined by means of the fluorescent lanthanide chelate label from the identified microparticle for the measurement of the labelled bioaffinity reactant; a different lanthanide from the one used for the identification measurement is now employed. In multiparameter assays particles with a diameter of < 1 mm are used as solid phase carriers.

The label(s) used for the identification of the microparticles can be linked to the microparticles during their manufacture by e.g. copolymerization. Alternatively, the label(s) can be chemically linked to the surface of the microparticles after the manufacturing process.

The present invention will be described in more detail by means of the following non-restrictive examples.

### Example 1

### Simultaneous assay of thyrotropin (TSH), thyroxine (T3), free thyroxine (T4) and thyroglobulin (TG) concentrations in blood samples

Multiparameter assay could be typically applied to the assessment of the thyroid function in the human by simultaneously measuring in e.g. a blood sample the concentrations of thyroptropin (TSH), thyroxine (T3), free thyroxine (T4) and thyroglobulin (TG). In this example four particle categories of identical size and properties, labelled for the purpose of the identification of the particle categories with different terbium chelate concentrations (1 x Tb, for the TSH assay; 5 x Tb, for the T3 assay; 10 x Tb, for the free T4 assay, and 15 x Tb, for the TG assay). During the production phase the particle categories are kept separate until it has been confirmed that they are functional in the said assays, at which point they are mixed for the multiparameter assay. TSH and TG assays are non-competitive while T3 and free T4 assays are competitive. 1 x Tb particles are coated with a monoclonal TSH- specific antibody, 5 x TB particles with an anti-T3 antibody, 10 x Tb particles with an anti- free T4 antibody, and 15 x Tb particles with a monoclonal TG-specific antibody, respectively. In the multiparameter assay the following bioaffinity reactants labelled with a fluorescent Eu chelate are used: Eu labelled monoclonal TSH-specific antibody, Eu labelled T3 derivative, Eu labelled T4 analogue, and Eu labelled monoclonal TG-specific antibody. In the multiparameter assay the mixture of the particle categories; the sample, and the reactants labelled with the Eu chelate are allowed to react simultaneously in a one-step assay. After incubation the particle categories are identified on the basis of their concentration of Tb chelate, and the Eu concentration of individual identified particles is measured. Time-resolved fluorescence is used for both identification and measurement. The concentration of each analyte (TSH, T3, free T4 and TG) is calculated from the measured Eu concentrations. In time-resolved fluorescence measurement either a flow cytometer, time-resolved microscope or time-resolved microfluorometer or other measuring instruments based on time-resolved technology are used (US 5,028,545; Xu, Y.Y. et al., Clin. Chem. 1992; 38/10: 2038 - 2043; Seveus, L. et al., Cytometry 1992; 13: 329 - 338).

### Example 2

### Simultaneous detection of a plurality of mutations

The multiparameter assay can be used for the diagnosis of an inherited disease by simultaneously detecting at the gene level a plurality of mutations associated with the disease. For instance, in the diagnosis of Duchenne muscular dystrophy (DMD) nine different deletion possibilities, all disease-associated, must be accurately identified in the gene. The multiparameter assay is performed in the following manner: the gene region to be examined is amplified by e.g. the PCR method (PCR = polymerase chain reaction), whereafter eighteen (nine normal and nine mutated alleles) particle categories identical in size and properties are used, which categories can be identified on the basis of the concentrations of the fluorescent Tb chelate they contain (1x, 2x, 4x, 8x, 16x, 32x, 64x, 128x, 256x, 521x, 1024x, 2048x, 4096x, 8192x, 16384x, 32768x, 65536x, 131072xTb). In the production step a nucleic acid probe (bioaffinity reactant) specifically identifying one of the aforementioned eighteen mutations, is immobilized onto the surface of each microparticle category. For the multiparameter assay a mixture is prepared from the particle categories, which mixture contains all eighteen particle categories required for the detection of the mutations in DMD. In the assay proper the said particle mixture, sample and nine different nucleic acid probes, all labelled with a fluorescent Eu chelate, are required. After incubation the category of individual particles is identified on the basis of the intensity of their Tb fluorescence, and the Eu concentration of individual particles is measured.

The multiparameter assay thus allows the detection of all known nine mutations associated with DMD. Time-resolved fluorescence measurements are performed with either a flow cytometer, time-resolved fluorescence microscope or microfluorometer (US 5,028,545; Xu, Y.Y. et al., Clin, Chem. 1992; 38/10: 2038 - 2043; Seveus, L. et al., Cytometry 1992; 13: 329 - 338).

A specialist in the field appreciates that the different applications of the said invention may vary within the scope of the claims. It will be appreciated that the methods of the present invention can be incorporated in the form of a variety of embodiments, only a few of which are disclosed herein. It will be apparent to the artisan that other embodiments exist. Thus, the described embodiments are illustrative and should not be construed as restrictive.

## Claims

1. A biospecific multiparameter assay method using microparticles distributed into different categories and representing different analytes, in which method microparticles belonging to different categories are labelled with a fluorescent molecule (1) and in which method the microparticles belonging to different categories are coated with bioaffinity reactants A binding different analytes, in which method
- microparticles belonging to different categories are mixed and the sample to be analyzed is added to the mixture, and bioaffinity reactants B labelled with a fluorescent molecule (2) are added to the mixture either immediately or after the completion of the reaction between the analyte molecule and the bioaffinity reactant A,
- the fluorescent molecules (1, 2) are excited, and the fluorescent emissions are quantitated for the identification of the microparticle category and for the measurement of the contents of different analytes,
**characterized** by
- the labelling of the microparticles and of the bioaffinity reactants B with different fluorescent molecules emitting long-lived fluorescence (1, 2).

2. The method according to claim 1, **characterized** by the use of two or more fluorescent molecules (1a, 1b...) emitting long-lived fluorescence for the identification of the microparticle category in such a manner that the different molecules (1a, 1b...) are located on the same or on different microparticles.

3. The method according to claim 1, **characterized** by the occurrence of at least one of the molecules emitting long-lived fluorescence (1, 1a, 1b...) in several concentrations on the microparticles.

4. The method according to claim 1, **characterized** by additionally labelling the microparticles with a molecule emitting short-lived fluorescence (3), which may occur on the same microparticles labelled with one or more molecules emitting long-lived fluorescence (1, 1a, 1b...) or on different microparticles.

5. The method according to claim 4, **characterized** by the use of the molecule emitting short-lived fluorescence, in different concentrations.

6. The method according to claims 1, **characterized** by Eu, Tb, Sm or Dy chelates being those molecules (1, 2) emitting long-lived fluorescence.

7. The method according to claims 1, **characterized** by the diameter of the microparticles being less than 1 mm.

## Patentansprüche

1. Biospezifisches Multiparameter-Assay-Verfahren unter Verwendung von Mikroteilchen, die in verschiedene Kategorien verteilt sind und verschiedene Analyten darstellen, wobei Mikropartikel, die verschiedenen Kategorien angehören, mit einem fluoreszierenden Molekül (1) markiert sind, und wobei die Mikropartikel, die verschiedenen Kategorien angehören, mit Bioaffinitätsreaktanten A, die verschiedene Analyten binden, beschichtet sind, wobei
- Mikropartikel, die veschiedenen Kategorien angehören, vermischt werden und die zu analysierende Probe dem Gemisch zugesetzt wird und Bioaffinitätsreaktanten B, die mit einem fluoreszierenden Molekül (2) markiert sind, dem Gemisch entweder sofort oder nach Beendigung der Reaktion zwischen dem Analytmolekül und dem Bioaffinitätsreaktanten A zugesetzt werden,
- die fluoreszierenden Moleküle (1, 2) angeregt werden und die Fluoreszenzemissionen quantitativ zur Identifikation der Mikroteilchenkategorie und zur Messung des Gehalts der verschiedenen Analyten bestimmt werden,
dadurch **gekennzeichnet**, daß
- die Mikropartikel und die Bioaffinitätsreaktanten B mit verschiedenen fluoreszierenden Molekülen, die eine langlebige Fluoreszenz emittieren (1, 2), markiert werden.

2. Verfahren nach Anspruch 1, **gekennzeichnet** durch die Verwendung von zwei oder mehr fluoreszierenden Molekülen (1a, 1b ...), die langlebige Fluoreszenz emittieren, zur Identifikation der Mikropartikelkategorie so, daß die verschiedenen Moleküle (1a, 1b...) auf dem gleichen oder auf verschiedenen Mikropartikeln lokalisiert werden.

3. Verfahren nach Anspruch 1, **gekennzeichnet** durch das Auftreten von mindestens einem der Moleküle, die eine langlebige Fluoreszenz emittieren (1, 1a, 1b..) in verschiedenen Konzentrationen auf den Mikropartikeln.

4. Verfahren nach Anspruch 1, dadurch **gekennzeichnet**, daß die Mikropartikel mit einem Molekül, das kurzlebige Fluoreszenz emittiert (3), zusätzlich markiert werden, das auf den gleichen Mikropartikeln, die mit einem oder mehreren Molekülen markiert sind, die langlebige Fluoreszenz (1, 1a, 1b ...) emittieren, oder auf verschiedenen Mikropartikeln vorkommen kann.

5. Verfahren nach Anspruch 4, dadurch **gekennzeichnet**, daß das Molekül, das eine kurzlebige Fluoreszenz emittiert, in verschiedenen Konzentrationen verwendet wird.

6. Verfahren nach Anspruch 1, dadurch **gekennzeichnet**, daß Eu-, Tb-, Sm- oder Dy-Chelate diejenigen Moleküle (1, 2) sind, die langlebige Fluoreszenz emittieren.

7. Verfahren nach Anspruch 1, dadurch **gekennzeichnet**, daß der Durchmesser der Mikropartikel weniger als 1 mm beträgt.

## Revendications

1. Procédé de dosage multiparamétrique biospécifique utilisant des microparticules distribuées dans différentes catégories et représentant différents analytes, procédé dans lequel les microparticules appartenant à différentes catégories sont marquées avec une molécule fluorescente (1) et procédé dans lequel les microparticules appartenant à différentes catégories sont revêtues de réactifs A à bioaffinité liant différents analytes, procédé dans lequel:
- des microparticules appartenant à différentes catégories sont mélangées et l'échantillon à analyser est ajouté au mélange, et les réactifs à bioaffinité B marqués avec une molécule fluorescente (2) sont ajoutés au mélange soit immédiatement, soit après l'achèvement de la réaction entre la molécule d'analyte et le réactif à bioaffinité A;
- les molécules fluorescentes (1, 2) sont excitées, et les émissions fluorescentes sont quantifiées pour l'identification de la catégorie des microparticules et pour la mesure des teneurs de différents analytes;
caractérisé par:
- le marquage des microparticules et des réactifs à bioaffinité B avec différentes molécules fluorescentes émettant une fluorescence à longue durée de vie (1, 2).

2. Procédé selon la revendication 1, caractérisé par l'utilisation de deux ou plusieurs molécules fluorescentes (1a, 1b...) émettant une fluorescence à longue durée de vie pour l'identification de la catégorie de microparticules d'une manière telle que les molécules différentes (1a, 1b...) sont situées sur les microparticules identiques ou différentes.

3. Procédé selon la revendication 1, caractérisé par l'occurrence d'au moins une des molécules émettant une fluorescence à longue durée de vie (1, 1a, 1b...) dans plusieurs concentrations sur les microparticules.

4. Procédé selon la revendication 1, caractérisé par le marquage supplémentaire des microparticules avec une molécule émettant une fluorescence à courte durée de vie (3), qui peuvent apparaître sur les mêmes microparticules marquées avec une ou plusieurs molécules émettant une fluorescence à longue durée de vie (1, 1a, 1b...) ou sur différentes microparticules.

5. Procédé selon la revendication 4, caractérisé par l'utilisation de la molécule émettant une fluorescence à courte durée de vie, dans différentes concentrations.

6. Procédé selon la revendication 1, caractérisé par des chélates de Eu, Tb, Sm ou Dy étant ces molécules (1, 2) émettant une fluorescence à longue durée de vie.

7. Procédé selon la revendication 1, caractérisé par le diamètre des microparticules étant inférieur à 1 mm.
